**Europäisches Patentamt**

**European Patent Office**

**Office européen des brevets**

(19)

(11) Veröffentlichungsnummer: **0 264 866**
**A2**

(12) **EUROPÄISCHE PATENTANMELDUNG**

(21) Anmeldenummer: 87115218.7

(22) Anmeldetag: **17.10.87**

(51) Int. Cl.⁴: **G01N 33/543** , G01N 33/569 , G01N 33/576

Patentanspruch für folgende Vertragsstaat: ES.

(30) Priorität: 24.10.86 DE 3636271
17.11.86 DE 3639279

(43) Veröffentlichungstag der Anmeldung:
**27.04.88 Patentblatt 88/17**

(84) Benannte Vertragsstaaten:
**AT BE CH DE ES FR GB IT LI LU NL SE**

(71) Anmelder: **BEHRINGWERKE**
**Aktiengesellschaft**
**Postfach 1140**
**D-3550 Marburg 1(DE)**

(72) Erfinder: **Dopatka, Hans-Detlef, Dr.**
**Heinrich-Heine-Strasse 9**
**D-3550 Marburg(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) **Verfahren zur Bestimmung des Vorhandenseins von diagnostisch relevanten Substanzen, insbesondere Antikörpern oder Antigenen nach der ELISA-Methode durch photometrische Auswertung.**

(57) Angegeben wird ein Verfahren zur Detektion und stufenlosen Quantifizierung von diagnostisch relevanten Substanzen, wobei ein einziger Probenansatz für die Untersuchung der Probe ausreicht. Als Test können ELISA, RIA, Nukleinsäure-Hybridisierung, Nephelometrie u. a. Bestimmungsmethoden verwendet werden. Als diagnostisch relevante Substanzen werden z. B. Antigene, Antikörper der verschiedenen Immunglobulinklassen, Nukleinsäuren oder Stoffwechselprodukte von medizinischer Bedeutung nachgewiesen. An einer Probe in der gewählten Testverdünnung mit eingefärbten enzymatisch markierten Antigen-/Antikörperkomplexen wird die Extinktion gemessen und aus dem erhaltenen Signal $A_{OD}$ der Titer rechnerisch nach der Formel:

$$\lg \text{Titer} = \text{alpha} \cdot A_{OD}^{\text{beta}}$$

ermittelt, der dem durch Verdünnungsreihen erhaltbaren Endverdünnungstiter mit guter Genauigkeit gleichgesetzt werden kann. Die Werte für alpha und beta werden in getrennten Versuchsreihen experimentell ermittelt.

FIG. 1

EP 0 264 866 A2

## Verfahren zur Bestimmung des Vorhandenseins von diagnostisch relevanten Substanzen, insbesondere Antikörpern oder Antigenen nach der ELISA-Methode durch photometrische Auswertung

Die Erfindung betrifft ein Verfahren zur Bestimmung des Vorhandenseins von diagnostisch relevanten Substanzen, insbesondere Antikörpern oder Antigenen, in Testflüssigkeiten, vorzugsweise nach der ELISA-Methode durch photometrische Auswertung der aus den Testflüssigkeiten hergestellten Proben mit eingefärbten enzymatisch markierten Antigen-/Antikörperkomplexen, bei dem nur eine einzige Testverdünnung der Probe hergestellt wird, in dieser Testverdünnung eine Antigen-/Antikörper-Bindungsreaktion ausgelöst wird, wobei vorzugsweise ein Reaktionspartner an einer festen Oberfläche fixiert ist, und der Antigen-Antikörperkomplex einer enzymatisch herbeigeführten Farbreaktion unterworfen wird, um eine Farblösung herzustellen, und aus der an dieser Farblösung gemessenen Extinktion unter Verwendung vorher gemessener Referenzdaten ein dem Endverdünnungstiter entsprechender Titer ermittelt wird.

Die Erfindung dient zur Detektion und stufenlosen Quantifizierung von diagnostisch relevanten Substanzen, wobei ein einziger Probenansatz für die Untersuchung der Probe ausreicht. Als Test können hierbei der Enzyme-Linked-Immunosorbent-Assay (ELISA), der Radio-Immuno-Assay (RIA), die Nukleinsäure-Hybridisierung, die Nephelometrie oder andere Bestimmungsmethoden verwendet werden. Als diagno stisch relevante Substanzen werden beispielsweise Antigene, Antikörper der verschiedenen Immunglobulinklassen, Nukleinsäuren oder auch Stoffwechselprodukte von medizinischer Bedeutung angesehen.

Die gängigen Quantifizierungsverfahren von diagnostisch relevanten Substanzen lassen sich auf zwei grundsätzliche Prinzipien zurückführen. Einmal kann die Quantifizierung der zu bestimmenden Substanz über eine Bezugskurve erfolgen, die vorher aus Proben mit bekanntem Substanzgehalt konstruiert wurde. Die alternative Möglichkeit besteht in einer Titration der zu bestimmenden Substanz mittels Endpunktverdünnung. Letzterer Weg empfiehlt sich immer dann, wenn bei unverdünnt oder nur schwach verdünnt zu testenden Proben die oben erwähnte Bezugskurve schwierig zu erstellen oder nur unbefriedigend zu nutzen ist. Dies ist beispielsweise bei der Bestimmung von testspezifischen Antikörpern im ELISA der Fall.

Aus diesem Grund wird das erfindungsgemäße Verfahren auch vorzugsweise in diesem Fall angewendet und in dieser Ausführungsform später näher erläutert. Als Beispiel für diagnostisch relevante Substanzen werden also Antikörper und als Beispiel für die eingangs erwähnten Tests der ELISA verwendet. Demzufolge bedeutet in der Beschreibung und in den Ansprüchen ein "spezifisches Farbsignal" sinnentsprechend für andere Tests beispielsweise auch radioaktive Zerfälle (counts) oder relatives Streulichtsignal.

Die ELISA-Methode ist ein bekannter Enzym-Immuntest, der zum Nachweis von Antikörpern oder Antigenen bei parasitären, bakteriellen oder viralen Infektionen dient. Die Erfindung dient zur Verbesserung der Auswertung von Farblösungen, die durch Inkontaktbringen beispielsweise des auf bestimmte Antikörper zu testenden Serums in einer speziellen Testverdünnung mit an Mikrotitrationsplatten fixierten Antigenen mit nachfolgendem enzymatischen Nachweis der gegebenenfalls entstandenen Antikörper-/Antigen-Komplexe gebildet werden. Insbesondere bezieht sich die Erfindung auf die photometrische Auswertung derartiger Lösungen, bei der die Extinktion oder optische Dichte der so hergestellten Lösung gemessen wird, welche ein Maß für die gebildeten, enzymatisch markierten farbbildenden Immunkomplexe in der Lösung ist. In der Regel wird ein Serum als positiv bewertet, wenn die Extinktion der wie beschrieben gebildeten Lösung einen bestimmten Grenz-oder Schwellenwert überschreitet. Diskussionen über diese Art der Auswertung finden sich z. B. in Immun. Infekt. 9, 33-39, 1981.

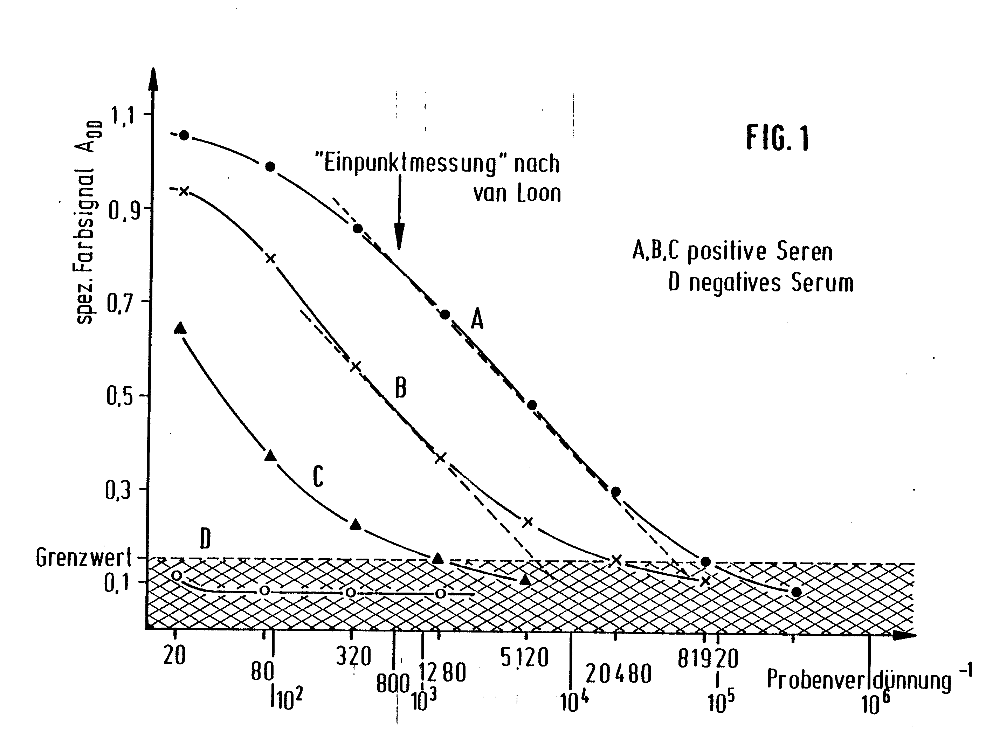

Es ist jedoch wünschenswert, nicht nur das Überschreiten oder Unterschreiten des Grenzwertes festzustellen, sondern auch quantitative Aussagen über das Vorhandensein von Antikörpern in dem zu testenden Serum machen zu können. Es ist bekannt, für diesen Zweck sogenannte "Verdünnungsreihen" durchzuführen, d. h. bei verschiedenen Probenverdünnungen den ELISA durchzuführen und die Extinktionen oder optischen Dichten der jeweils resultierenden Farblösungen zu messen. Durch Verbinden der einzelnen, photometrischen Meßwerte in einer Graphik ergibt sich für jede Serumprobe eine sogenannte Probenverdünnungskurve (Fig. 1). Diejenige Probenverdünnung, bei der die Probenverdünnungskurve den vorgegebenen Grenzwert schneidet, gibt für den zu bestimmenden Antikörper die Endverdünnung an, wobei diese Endverdünnung der reziproke Wert des Antikörpertiters ist (Endverdünnungstiter). Die Quantifizierung des Antikörpers erfolgt also über die Angabe der Probenverdünnung, in der die Nachweisgrenze erreicht wird.

Für die Meßpunkte der Probenverdünnungskurve werden häufig die gemessenen Extinktionswerte oder optische Dichten nicht im direkt gemessenen Zahlenwert verwendet, sondern es erfolgt meist eine Meßwertkorrektur. Die Art der Korrektur kann in der Subtraktion des Meßwertes einer im ELISA mitgeführten, geeigneten Kontrolle (Puffer, negatives Serum) oder eines Parallelansatzes des zu bestimmenden Serums mit an der Mikrotitrationsplatte fixiertem Kontrollantigen oder einer Startextinktion bei Beobachtung einer Farbentwicklungskinetik bestehen. Weiterhin· sind Multiplikationen des direkt gemessenen Meßwertes mit einem Korrekturfaktor, der sich in geeigneter Weise aus einem mitgeführten Standard ergibt, oder auch die Angabe eines Quotienten bzw. Index möglich, der sich durch Division des direkt gemessenen Meßwertes mit dem einer mitgeführten, negativen Probe ergibt. Dieser korrigierte Meßwert wird im weiteren als spezifisches Farbsignal bezeichnet und liegt der Angabe des Endverdünnungstiters zugrunde.

Der wesentliche Nachteil einer quantitativen Auswertung auf die beschriebene Weise ist, daß die Durchführung zeit-und reagenzienaufwendig ist. Es wurden deshalb Versuche unternommen, aus der an einer einzigen Testverdünnung im ELISA gemessenen Extinktion auf den Endverdünnungstiter des Serums zu schließen. So beschrieben beispielsweise van Loon und van der Veen in J. Clin. Pathol. 33, 635-639, 1980, den Nachweis von Toxoplasma-Antikörpern mit der ELISA-Methode unter Verwendung einer einzigen Serumverdünnung in Verbindung mit einer Referenzkurve. Dabei wird die Extinktion, die im ELISA bei einer Serumverdünnung von 1:800 gemessen wurde, mit der gleichen Extinktion in der Referenzkurve verglichen, welche die an einer Reihe von Seren in der Testverdünnung 1:800 gefundene Extinktion als Funktion des Endverdün nungstiters angibt. Dabei wurde diese Referenzkurve graphisch als Mittelwert der Meßwerte an einer Vielzahl von Seren konstruiert. Eine Verbesserung dieses Verfahrens wird von van Loon et al. in J. Clin. Pathol. 34, 1981, 665-669, beschrieben, bei der die Extinktionswerte durch die spezifischen Farbsignale ersetzt werden, welche durch Subtrahieren der Extinktion eines auf 1:800 verdünnten negativen Kontrollserums von der Extinktion des Testserums erhalten werden. Auf diese Weise konnten die Abweichungen experimentell vermessener Seren von der Referenzkurve zumindest in einigen Fällen verbessert werden. In anderen Fällen war die Verbesserung jedoch so gering, daß der Vergleich mit einem negativen Kontrollserum unnötig war.

Obgleich durch dieses bekannte Verfahren für die Praxis bereits schon eine Vereinfachung erreicht worden war, indem nämlich nur noch eine einzige Testverdünnung hergestellt werden muß, weist dieses Verfahren doch erhebliche Nachteile auf. Die für dieses Verfahren geeignete und empfohlene Testverdünnung von 1:800 ist sehr hoch, so daß sowohl die Empfindlichkeit des Verfahrens zu gering wird (siehe schwach positives Serum in Figur 1) als auch Pipettierungsfehler sich potenzieren. Außerdem ist in der Praxis das Arbeiten mit einer Referenzkurve lästig oder erfordert bei automatischer Meßdatenauswertung einen Computer.

Aufgabe der Erfindung ist es, ein Verfahren zur quantitativen Bestimmung von Antikörpern oder Antigenen z. B. im ELISA zu schaffen, bei dem eine möglichst geringe Verdünnung der zu untersuchenden Probe verwendet werden kann und die Titerbestimmung in einfacher Weise, z. B. mittels eines Taschenrechners durchgeführt werden kann.

Außerdem sollen Anwendungsgebiete für das Verfahren gemäß der Erfindung angegeben werden.

Gelöst wird diese Aufgabe durch ein Verfahren der eingangs angegebenen Art dadurch, daß die Extinktion (oder optische Dichte) der Farblösung gemessen wird und aus dem erhaltenen Signal $A_{OD}$ der Titer rechnerisch nach der Formel:

$$1g\ \text{Titer} = \text{alpha} \cdot A_{OD}^{beta} \qquad (1)$$

ermittelt wird, der dem durch Verdünnungsreihen erhaltbaren Endverdünnungstiter gleichgesetzt wird, wobei in der Formel (1) der "Titer" der reziproke Wert derjenigen Endverdünnung ist, bei der das Signal $A_{OD}$ dem Grenzwertsignal an der Nachweisgrenze gegenüber negativen Kontrollproben entspricht, und die Werte für alpha und beta bei festgelegter Testverdünnung für die jeweilige Kombination von immunologisch reaktiver Oberfläche und enzymmarkiertem Immunglobulin bzw. Antigen als Detektor bei gleichen Umsetzungs-und Fixierungsbedingungen getrennt experimentell durch Versuchsreihen an Proben mit bekanntem Endverdünnungstiter des Analyten bestimmt werden.

Vorzugsweise wird bei dem Verfahren als Signal $A_{OD}$ für die Extinktion das vorstehend definierte spezifische Farbsignal in die Formel (1) eingesetzt.

Als Testverdünnungen können Probenverdünnungen im Bereich von unverdünnt bis 1:800 verwendet werden, wobei einer Testverdünnung von nur 1:150 der Vorzug zu geben ist. Im letzteren Fall ist die Testverdünnung noch so gering, daß eine hohe Empfindlichkeit des Immuntestverfahrens gewährleistet ist, zum anderen haben die Experimente gezeigt, daß mit einer Testverdünnung von 1:150 relativ gut reproduzierbare Ergebnisse erhalten werden können.

Niedrigere Testverdünnungen sind möglich, erfordern aber sowohl bei der immunologisch reaktiven Oberfläche z. B. der antigenbeschichteten Mikrotitrationsplatte, als auch dem Konjugat einen hohen Reinheitsgrad bei hoher Reaktivität.

Es hat sich gezeigt, daß brauchbare Werte für alpha im Bereich von 3,0 bis 3,6 und für beta im Bereich von 0,10 bis 0,27 liegen.

Die richtige Bestimmung dieser Werte alpha und beta bildet die Grundlage für die vorteilhafte Anwendung des erfindungsgemäßen Verfahrens in der Praxis. Sobald einmal optimale Werte für alpha und beta gefunden sind, kann die Auswertung, d. h. die Titerbestimmung der Testprobe rechnerisch sehr einfach durchgeführt werden. Die Werte für alpha und beta können z. B. durch den Reagenzienhersteller anhand von Versuchsreihen an einer Vielzahl von Untersuchungsproben bestimmt werden, indem an diesen Seren jeweils das spezifische Farbsignal bei der gewünschten Testverdünnung gemessen und zusätzlich der Titer in an sich bekannter Weise durch Endpunktverdünnung bestimmt wird. Aus dem für jede Probe erhaltenen Wertepaar werden dann durch iterative Anpassung geeignete Werte für alpha und beta gesucht, für die die Übereinstimmung zwischen dem durch Endpunktverdünnung gemessenen Titer $T_{mess}$ und dem aus der Formel (1) nach Einsetzen des spezifischen Farbsignals errechneten Titer $T_{rech}$ ein Optimum erreicht wird. Bei einem derartigen iterativen Verfahren werden beispielsweise mit zunächst willkürlich angenommenen Zahlenwerten für alpha und beta über Formel (1) aus dem spezifischen Farbsignal jeder Probe die zugehörigen Titer $T_{rech}$ errechnet. Außerdem wird durch Endpunktverdünnung der Titer $T_{mess}$ bestimmt.

Wenn der Quotient $T_{mess}/T_{rech}$ = 1,0 ist, würde dies bedeuten, daß der richtige Titer aus der Extinktionsmessung bei der Testverdünnung abgeleitet worden ist. Unter realistischen Bedingungen muß ein Quotient zwischen 0,8 und 1,25 als "Titer exakt getroffen" angesehen werden. Quotienten unter 0,5 und über 2,0 bedeuten, daß die für eine klassische Titration zulässigen Reproduzierbarkeitsgrenzen überschritten wurden.

Nach dem ersten Versuch, der üblicherweise eine noch schlechte rechnerische Titervorhersage liefert, werden dann alpha und beta schrittweise verändert und danach erneut für jede Serumprobe der errechnete Titer mit dem gemessenen Titer verglichen. Dieses Iterationsverfahren wird fortgesetzt, bis die Übereinstimmung zufriedenstellend ist.

Die Versuche zeigten, daß davon ausgegangen werden kann, daß das Optimum erreicht ist, wenn in einer Versuchsreihe für etwa 50 % der Seren gilt

$$0,8 \le T_{mess}/T_{rech} \le 1,25. \qquad (2)$$

Diese Erfahrungen beziehen sich auf Testreihen mit alkalischer Phosphatase als Indikatorenzym. Wenn Peroxidase als Indikatorenzym eingesetzt werden kann, kann die "Trefferrate" auf etwa 65 % gesteigert werden. Diese Grenzen liegen jedoch im Bereich der Streuungen der ELISA-Methode selbst und können somit durch das erfindungsgemäße Verfahren zur quantitativen Auswertung der Meßergebnisse nicht überwunden werden.

Weiterhin hat sich gezeigt, daß die Werte von alpha und beta nicht nur von der Art des diagnostischen Tests, sondern auch von den Herstellungschargen der eingesetzten, immunologisch reaktiven festen Phase und des Konjugats abhängen. Die beiden Konstanten müssen dementspre chend für den praktischen Einsatz des Verfahrens z. B. jeweils für die Kombination einer Testplatten-und einer Konjugatcharge neu bestimmt werden, gestatten dann aber Reihenmessungen an Proben, die untersucht werden sollen mit guter Reproduzierbarkeit und für die Praxis sehr guter Genauigkeit.

Im folgenden wird die Erfindung anhand von Beispielen und Meßergebnissen näher erläutert. Dabei wird auch auf die beigefügten Zeichnungen Bezug genommen.

In den Zeichnungen zeigen:

Figur 1 gemäß dem Stand der Technik durchgeführte Verdünnungsreihen in Form von Probenverdünnungskurven, die das spezifische Farbsignal als Funktion der Probenverdünnungen zeigen,

Figur 2 die Abhängigkeit des spezifischen Farbsignals bei einer Probenverdünnung von 1:150 von dem tatsächlichen, durch Endverdünnung bestimmten Titer ($T_{mess}$),

Figur 3 die Abhängigkeit des nach der Formel (1) errechneten Titers ($T_{rech}$) vom spezifischen Farbsignal bei einer Probenverdünnung von 1:150 an den gleichen Testreagenzien wie in Figur 2 und

Figur 4 den gemessenen Titer $T_{mess}$ aus Figur 2 im Vergleich zu dem errechneten Titer $T_{rech}$ aus Figur 3 bei identischem spezifischem Farbsignal der 1:150 verdünnten Untersuchungsproben.

Zur Prüfung des Verfahrens gemäß der Erfindung wurden Versuche zur Bestimmung von Antikörpern gegen Cytomegalovirus in Seren mittels Mikrotitrationsplatten, an deren Wänden Cytomegalovirus-Antigen fixiert war, und zur Bestimmung von Antikörpern gegen Rubella in Seren mittels Mikrotitrationsplatten, an deren Wand Rubella-Antigen fixiert war, durchgeführt. Die Untersuchungsproben in der Testverdünnung 1:150 wurden zur Reaktion mit den fixierten Antigenen gebracht, die nicht gebundenen Antikörper ausgewaschen, über eine Konjugatlösung ein Enzym an den gebildeten Antikörper-/Antigen-Komplexen gebunden, die überschüssige Konjugatlösung ausgewaschen, mittels einer chromogenen Substratlösung die Einfärbung über das Enzym bewirkt und anschließend mit einer Stop-Lösung die Reaktion beendet. Die so hergestellten, in Abhängigkeit vom Gehalt der virusspezifischen Antikörper eingefärbten Proben wurden dann bewertet, indem ihre optische Dichte oder· Extinktion $A_{OD}$ photometrisch (bei einer definierten Wellenlänge) bestimmt wurde.

In Verdünnungsreihen wurde bei denselben Proben zum einen der Endverdünnungstiter $T_{mess}$ in an sich bekannter Weise bestimmt und mit dem über die Formel (1) gemäß der Erfindung berechneten Titer $T_{rech}$ verglichen.

Zur Verbesserung der Reproduzierbarkeit und der Genauigkeit des Verfahrens wurden in allen Fällen die spezifische Extinktion oder das "spezifische Farbsignal $A_{OD}$" verwendet.

Figur 1 zeigt eine Kurvendarstellung des spezifischen Farbsignals $A_{OD}$ in Abhängigkeit von verschiedenen Probenverdünnungen (Probenverdünnungen$^{-1}$) für drei positive Seren A, B und C und für ein negatives Serum D. Die exemplarischen Darstellungen zeigen Kurvenverläufe, die nur in Bereichen höherer Testverdünnungen, beispielsweise 1:800, zu parallelisieren sind. Hierauf beruht die Erstellung einer geeigneten Referenzkurve nach van Loon.

Während das Abknicken der Probenverdünnungskurve bei einem Serum mit hohem Antikörpertiter (Beispiel A) den literaturbekannten Prozoneneffekt darstellt, ist es nicht ganz klar, warum die Verdünnungskurven im unteren Bereich in der Nähe des Endverdünnungstiters abgeflacht werden.

Es wurde nun gefunden, daß der durch eine Verdünnungsreihe bestimmte Endverdünnungstiter an einer breiten Vielfalt von Proben zu der bei einer Testverdünnung von z. B. 1:150 gemessenen Extinktion in der Beziehung steht:

$$\lg \text{Titer} = \text{alpha} \cdot A_{OD}^{\text{beta}} \qquad (1)$$

wobei die Konstanten alpha und beta eine Anpassung an die Proben-und Testbedingungen gestatten.

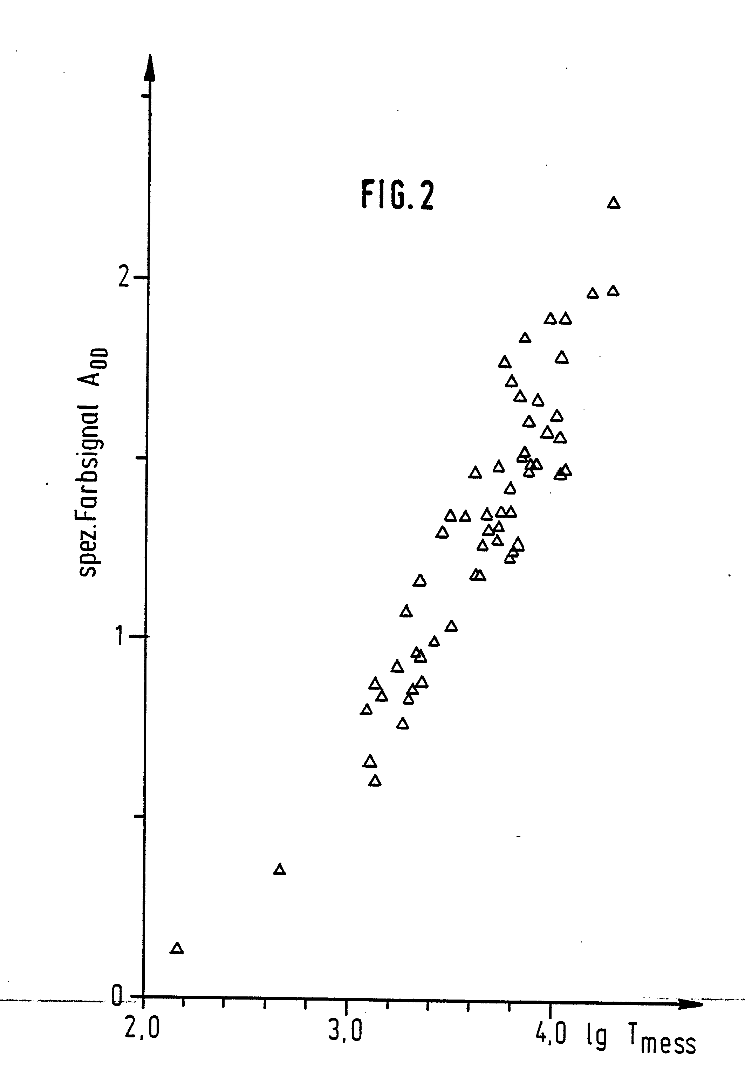

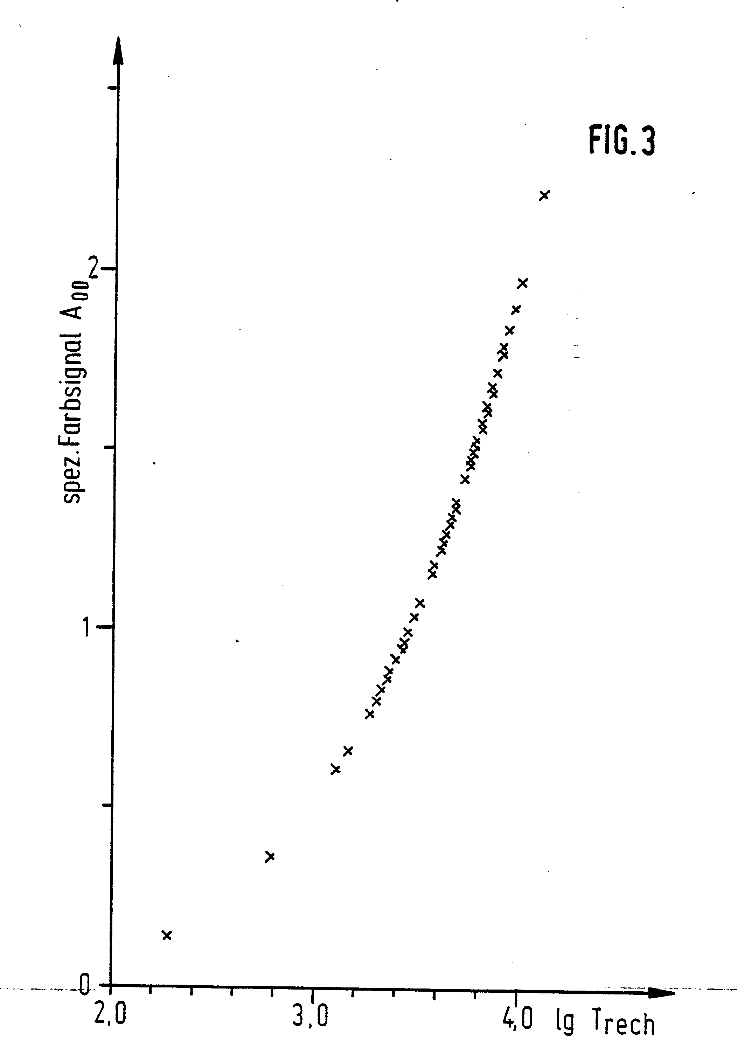

Diese Tatsache ergibt sich aus den Figuren 2 bis 4. In Figur 2 ist die Abhängigkeit des spezifischen Farbsignals $A_{OD}$ von 58 Seren in einer .Verdünnung von 1:150 als Funktion des durch Endverdünnung bestimmten Titers $T_{mess}$ aufgetragen. Andererseits zeigt Figur 3 den Titer, der nach Formel (1) aus der Extinktion $A_{OD}$ bei optimalen Werten für alpha und beta berechnet worden ist. Die geringe Streubreite dieser Kurve ergibt sich aus der mathematischen Erstellung.

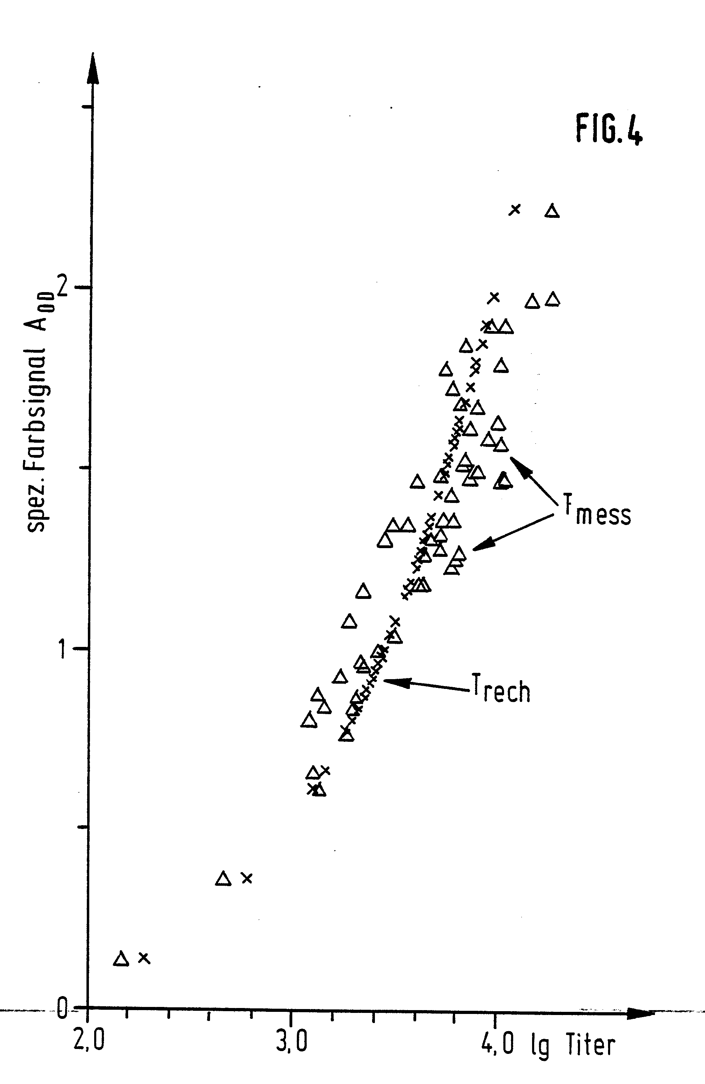

Figur 4 zeigt nun deutlich, daß die nach Formel (1) errechnete Kurve der Titerwerte die Meßwerte hervorragend mittelt. Hieraus ergibt sich somit, daß mit dem Verfahren der Erfindung eine optimale Voraussage der Endverdünnungstiter erzielt werden kann.

Diese gute Übereinstimmung zwischen $T_{rech}$ und $T_{mess}$ wird allerdings nur dann erhalten, wenn Sorgfalt auf die Bestimmung der Werte für alpha und beta gelegt wird.

Anhand der folgenden Tabelle von gemessenen und rechnerisch ermittelten Daten einer Versuchsreihe an 60 Seren wird nun als Beispiel das Vorgehen zur Bestimmung und Optimierung von alpha und beta erläutert.

## TABELLE 1

| spez. Farbsignal bei 1:150 | $T_{mess}$ | $T_{rech}$ | $T_{mess}$ / $T_{rech}$ kleiner als 0,8 | im Bereich 0,8-1,25 | größer als 1,25 |
|---|---|---|---|---|---|
| 0.361 | 1: 466 | 1: 610 | 0.763 | | |
| 0.606 | 1: 1372 | 1: 1285 | | 1.068 | |
| 0.660 | 1: 1247 | 1: 1464 | | 0.852 | |
| 0.768 | 1: 1862 | 1: 1857 | | 1.003 | |
| 0.805 | 1: 1225 | 1: 2002 | 0.612 | | |
| 0.838 | 1: 1985 | 1: 2137 | | 0.929 | |
| 0.843 | 1: 1446 | 1: 2157 | 0.67 | | |
| 0.865 | 1: 2058 | 1: 2250 | | 0.915 | |
| 0.879 | 1: 1348 | 1: 2310 | 0.584 | | |
| 0.886 | 1: 2298 | 1: 2340 | | 0.982 | |
| 0.928 | 1: 1730 | 1: 2526 | 0.685 | | |
| 0.958 | 1: 2264 | 1: 2664 | | 0.85 | |
| 0.968 | 1: 2166 | 1: 2710 | 0.799 | | |
| 0.999 | 1: 2622 | 1: 2858 | | 0.918 | |
| 1.041 | 1: 3185 | 1: 3064 | | 1.039 | |
| 1.081 | 1: 1911 | 1: 3268 | 0.585 | | |
| 1.166 | 1: 2230 | 1: 3725 | 0.599 | | |
| 1.184 | 1: 4410 | 1: 3825 | | 1.153 | |
| 1.185 | 1: 4165 | 1: 3831 | | 1.087 | |
| 1.202 | 1: 1127 | 1: 3928 | 0.287 | | |
| 1.233 | 1: 6096 | 1: 4108 | | | 1.484 |
| 1.255 | 1: 6321 | 1: 4238 | | | 1.492 |
| 1.266 | 1: 4459 | 1: 4304 | | 1.036 | |
| 1.270 | 1: 6591 | 1: 4328 | | | 1.523 |
| 1.281 | 1: 5317 | 1: 4395 | | 1.21 | |
| 1.305 | 1: 2842 | 1: 4543 | 0.626 | | |
| 1.312 | 1: 4802 | 1: 4586 | | 1.047 | |
| 1.321 | 1: 5390 | 1: 4643 | | 1.161 | |
| 1.348 | 1: 3700 | 1: 4814 | 0.769 | | |

6

| spez. Farbsignal bei 1:150 | $T_{mess}$ | $T_{rech}$ | $T_{mess}$ / $T_{rech}$ kleiner als 0,8 | im Bereich 0,8-1,25 | größer als 1,25 |
|---|---|---|---|---|---|
| 1.349 | 1: 3112 | 1: 4820 | 0.646 | | |
| 1.354 | 1: 4753 | 1: 4853 | | 0.979 | |
| 1.363 | 1: 5537 | 1: 4911 | | 1.128 | |
| 1.364 | 1: 6223 | 1: 4917 | | | 1.266 |
| 1.429 | 1: 6076 | 1: 5349 | | 1.136 | |
| 1.470 | 1: 4116 | 1: 5633 | 0.731 | | |
| 1.471 | 1:10682 | 1: 5640 | | | 1.894 |
| 1.476 | 1: 7473 | 1: 5675 | | | 1.317 |
| 1.477 | 1:11172 | 1: 5682 | | | 1.966 |
| 1.486 | 1: 5341 | 1: 5746 | | 0.93 | |
| 1.496 | 1: 7718 | 1: 5817 | | | 1.327 |
| 1.496 | 1: 8232 | 1: 5817 | | | 1.415 |
| 1.515 | 1: 6983 | 1: 5953 | | 1.173 | |
| 1.537 | 1:10633 | 1: 6114 | | | 1.739 |
| 1.586 | 1: 9212 | 1: 6480 | | | 1.422 |
| 1.616 | 1: 7473 | 1: 6710 | | 1.114 | |
| 1.632 | 1:10266 | 1: 6835 | | | 1.502 |
| 1.674 | 1: 8134 | 1: 7168 | | 1.135 | |
| 1.684 | 1: 6689 | 1: 7249 | | 0.923 | |
| 1.727 | 1: 6101 | 1: 7603 | | 0.802 | |
| 1.747 | 1:20355 | 1: 7770 | | | 2.62 |
| 1.780 | 1: 5635 | 1: 8052 | 0.7 | | |
| 1.793 | 1:10633 | 1: 8164 | | | 1.302 |
| 1.846 | 1: 7032 | 1: 8633 | | 0.815 | |
| 1.898 | 1: 9408 | 1: 9107 | | 1.033 | |
| 1.901 | 1:11074 | 1: 9135 | | 1.212 | |
| 1.937 | 1:14945 | 1: 9473 | | | 1.578 |
| 1.980 | 1:18718 | 1: 9886 | | | 1.893 |
| 2.012 | 1:21805 | 1:10200 | | | 2.138 |
| 2.211 | 1:25725 | 1:12289 | | | 2.093 |
| 2.224 | 1:18375 | 1:12433 | | | 1.478 |

In Spalte 1 der Tabelle sind Meßwerte des spezifischen Farbsignals an Proben in einer Testverdünnung von 1:150 für 60 Seren angegeben. In Spalte 2 ist der durch Endverdünnungsreihen gemessene Titer $T_{mess}$ für die gleichen Seren angegeben. Spalte 3 zeigt für die gleichen Seren jeweils den nach Formel (1) aus dem Farbsignal der Spalte 1 unter Verwendung der nach Optimierung gefundenen Werte alpha = 3,4514 und beta = 0,2106 berechneten Titer $T_{rech}$.

Mit Hilfe moderner zur Verfügung stehender Rechner bedeutet es heute keinen hohen Aufwand, das angewendete iterative Verfahren zur Optimierung von alpha und beta für bestimmte Reagenzienkombinationen von einem Fachmann programmieren zu lassen.

Der Quotient $T_{mess}/T_{rech}$ ist nachfolgend angegeben, und zwar gewichtet nach "zu klein" (kleiner als 0,8), "etwa gleich 1" (im Bereich von 0,8 - 1,25) und "zu groß" (größer als 1,25).

In dem dargestellten Fall ist ersichtlich, daß für 45 % der Seren eine gute Näherung erzielt worden ist. Unter Berücksichtigung der Streubreite der Endverdünnungstiter, die aus Figur 2 ersichtlich ist, kann deshalb die Wahl der Werte für alpha und beta als für die Praxis sehr gut angesehen werden.

## Ansprüche

1. Verfahren zur Bestimmung des Vorhandenseins von diagnostisch relevanten Substanzen, insbesondere Antikörpern oder Antigenen, in Testflüssigkeiten, vorzugsweise nach der ELISA-Methode durch photometrische Auswertung der aus den Testflüssigkeiten hergestellten Proben mit eingefärbten enzymatisch markierten Antigen-/Antikörperkomplexen, bei dem nur eine einzige Testverdünnung der Probe hergestellt wird, in dieser Testverdünnung eine Antigen/Antikörper-Bindungsreaktion ausgelöst wird, wobei vorzugsweise ein Reaktionspartner an einer festen Oberfläche fixiert ist, und der Antigen-/Antikörperkomplex einer enzymatisch herbeigeführten Farbreaktion unterworfen wird, um eine Farblösung herzustellen, und aus der an dieser Farblösung gemessenen Extinktion unter Verwendung vorher gemessener Referenzdaten ein dem Endverdünnungstiter entsprechender Titer ermittelt wird, dadurch gekennzeichnet, daß die Extinktion (oder optische Dichte) der Farblösung gemessen wird und aus dem erhaltenen Signal $A_{OD}$ der Titer rechnerisch nach der Formel:

$$1g \ Titer = alpha \cdot A_{OD}^{beta} \qquad (1)$$

ermittelt wird, der dem durch Verdünnungsreihen erhaltbaren Endverdünnungstiter gleichgesetzt wird, wobei in der Formel (1) der "Titer" der reziproke Wert derjenigen Endverdünnung ist, bei der das Signal $A_{OD}$ dem Grenzwertsignal an der Nachweisgrenze gegenüber negativen Kontrollproben entspricht, und die Werte für alpha und beta bei festgelegter Testverdünnung für die jeweilige Kombination von immunologisch reaktiver Oberfläche und enzymmarkiertem Immunglobulin bzw. Antigen als Detektor bei gleichen Umsetzungs-und Fixierungsbedingungen getrennt experimentell durch Versuchsreihen an Proben mit bekanntem Endverdünnungstiter des Analyten bestimmt werden.

2. Verfahren nach Anspruch 1, dadurch gekennzeichnet, daß als Signal $A_{OD}$ für die Extinktion oder optische Dichte das spezifische Farbsignal in die Formel (1) eingesetzt wird, das

a) durch Subtrahieren des Extinktionssignals einer im ELISA mitgeführten Kontrolle (z. B. Puffer oder negative Probe) oder des Extinktionssignals eines Parallelansatzes der zu untersuchenden Probe auf einer mit einer Kontrollpräparation beschichteten Oberfläche oder des Extinktionssignals als Beginn einer Farbzunahmemessung von dem Extinktionssignal der zu untersuchenden Probe,

b) durch Multiplizieren des Extinktionssignals der zu untersuchenden Probe mit einem sich aus einer mitgeführten Versuchskontrolle (z. B. Standard) ergebenden Korrekturfaktor, oder

c) nach Quotienten-bzw. Indexbildung aus dem Extinktionssignal der zu untersuchenden Probe und dem einer mitgeführten Versuchskontrolle (z. B. negative Probe)

erhalten worden ist.

3. Verfahren nach Anspruch 1 oder 2, dadurch gekennzeichnet, daß als Testverdünnungen Probenverdünnungen im Bereich von unverdünnt bis 1:800, vorzugsweise von etwa 1:150, verwendet werden.

4. Verfahren nach Anspruch 1, 2 oder 3, dadurch gekennzeichnet, daß für alpha Werte im Bereich von 3,0 bis 3,6 und für beta im Bereich von 0,10 bis 0,27 verwendet werden.

5. Verfahren nach Anspruch 1, 2 oder 3, dadurch gekennzeichnet, daß die Werte für alpha und beta durch Versuchsreihen an einer Vielzahl von Testflüssigkeiten oder Proben bestimmt werden, indem an diesen Proben jeweils das Signal $A_{OD}$ bei der gewünschten Testverdünnung gemessen und außerdem der Titer in an sich bekannter Weise durch Endpunktverdünnung bestimmt wird und dann aus den erhaltenen Wertepaaren (Titer, $A_{OD}$) für die einzelnen Testflüssigkeiten oder Proben durch iterative Anpassung geeignete Werte für alpha und beta gesucht werden, für die die Übereinstimmung zwischen dem durch Endpunktverdünnung gemessenen Titer ($T_{mess}$) und dem aus der Formel (1) durch Einsetzen des Signals $A_{OD}$ errechneten Titer ($T_{rech}$) ein Optimum erreicht.

6. Verfahren nach Anspruch 5, dadurch gekennzeichnet, daß iterativ das Wertepaar für alpha und beta ermittelt wird, bei dem der jeweilige Quotient $T_{mess}/T_{rech}$ der einzelnen Testflüssigkeiten oder Proben am häufigsten im Bereich von 0,8 bis 1,25 liegt.

7. Verwendung des Verfahrens nach einem der Ansprüche 1 bis 6 zur Bestimmung des Vorhandenseins von Rubella (IgG; IgM)-Antikörpern, Cytomegalovirus (IgG; IgM)-Antikörpern oder Hepatitis B-Oberflächen-Antigen ($HB_sAg$) in Seren mittels Mikrotitrationsplatten, an denen entsprechend Rubella-Antigen, Cytomegalovirus-Antigen oder Antikörper gegen $HB_sAg$ fixiert sind.

8. Verwendung nach Anspruch 7, dadurch gekennzeichnet, das alkalische Phosphatase oder Peroxidase als Indikatorenzym eingesetzt wird.

Patentansprüche für folgenden Vertragsstaat : ES

1. Verfahren zur Bestimmung des Vorhandenseins von diagnostisch relevanten Substanzen, insbesondere Antikörpern oder Antigenen, in Testflüssigkeiten, vorzugsweise nach der ELISA-Methode durch photometrische Auswertung der aus den Testflüssigkeiten hergestellten Proben mit eingefärbten enzymatisch markierten Antigen-/Antikörperkomplexen, bei dem nur eine einzige Testverdünnung der Probe hergestellt wird, in dieser Testverdünnung eine Antigen/Antikörper-Bindungsreaktion ausgelöst wird, wobei vorzugsweise ein Reaktionspartner an einer festen Oberfläche fixiert ist, und der Antigen-/Antikörperkomplex einer enzymatisch herbeigeführten Farbreaktion unterworfen wird, um eine Farblösung herzustellen, und aus der an dieser Farblösung gemessenen Extinktion unter Verwendung vorher gemessener Referenzdaten ein dem Endverdünnungstiter entsprechender Titer ermittelt wird, dadurch gekennzeichnet, daß die Extinktion (oder optische Dichte) der Farblösung gemessen wird und aus dem erhaltenen Signal $A_{OD}$ der Titer rechnerisch nach der Formel:

$$\lg \text{Titer} = \text{alpha} \cdot A_{OD}^{\text{beta}} \qquad (1)$$

ermittelt wird, der dem durch Verdünnungsreihen erhaltbaren Endverdünnungstiter gleichgesetzt wird, wobei in der Formel (1) der "Titer" der reziproke Wert derjenigen Endverdünnung ist, bei der das Signal $A_{OD}$ dem Grenzwertsignal an der Nachweisgrenze gegenüber negativen Kontrollproben entspricht, und die Werte für alpha und beta bei festgelegter Testverdünnung für die jeweilige Kombination von immunologisch reaktiver Oberfläche und enzymmarkiertem Immunglobulin bzw. Antigen als Detektor bei gleichen Umsetzungs- und Fixierungsbedingungen getrennt experimentell durch Versuchsreihen an Proben mit bekanntem Endverdünnungstiter des Analyten bestimmt werden.

2. Verfahren nach Anspruch 1, dadurch gekennzeichnet, daß als Signal $A_{OD}$ für die Extinktion oder optische Dichte das spezifische Farbsignal in die Formel (1) eingesetzt wird, das

a) durch Subtrahieren des Extinktionssignals einer im ELISA mitgeführten Kontrolle (z. B. Puffer oder negative Probe) oder des Extinktionssignals eines Parallelansatzes der zu untersuchenden Probe auf einer mit einer Kontrollpräparation beschichteten Oberfläche oder des Extinktionssignals als Beginn einer Farbzunahmemessung von dem Extinktionssignal der zu untersuchenden Probe,

b) durch Multiplizieren des Extinktionssignals der zu untersuchenden Probe mit einem sich aus einer mitgeführten Versuchskontrolle (z. B. Standard) ergebenden Korrekturfaktor, oder

c) nach Quotienten- bzw. Indexbildung aus dem Extinktionssignal der zu untersuchenden Probe und dem einer mitgeführten Versuchskontrolle (z. B. negative Probe) erhalten worden ist.

3. Verfahren nach Anspruch 1 oder 2, dadurch gekennzeichnet, daß als Testverdünnungen Probenverdünnungen im Bereich von unverdünnt bis 1:800, vorzugsweise von etwa 1:150, verwendet werden.

4. Verfahren nach Anspruch 1, 2 oder 3, dadurch gekennzeichnet, daß für alpha Werte im Bereich von 3,0 bis 3,6 und für beta im Bereich von 0,10 bis 0,27 verwendet werden.

5. Verfahren nach Anspruch 1, 2 oder 3, dadurch gekennzeichnet, daß die Werte für alpha und beta durch Versuchsreihen an einer Vielzahl von Testflüssigkeiten oder Proben bestimmt werden, indem an diesen Proben jeweils das Signal $A_{OD}$ bei der gewünschten Testverdünnung gemessen und außerdem der Titer in an sich bekannter Weise durch Endpunktverdünnung bestimmt wird und dann aus den erhaltenen Wertepaaren (Titer, $A_{OD}$) für die einzelnen Testflüssigkeiten oder Proben durch iterative Anpassung geeignete Werte für alpha und beta gesucht werden, für die die Übereinstimmung zwischen dem durch Endpunktverdünnung gemessenen Titer ($T_{mess}$) und dem aus der Formel (1) durch Einsetzen des Signals $A_{OD}$ errechneten Titer ($T_{rech}$) ein Optimum erreicht.

6. Verfahren nach Anspruch 5, dadurch gekennzeichnet, daß iterativ das Wertepaar für alpha und beta ermittelt wird, bei dem der jeweilige Quotient $T_{mess}/T_{rech}$ der einzelnen Testflüssigkeiten oder Proben am häufigsten im Bereich von 0,8 bis 1,25 liegt.

7. Verfahren nach einem der Ansprüche 1 bis 6, worin das Vorhandensein von Rubella (IgG; IgM)-Antikörpern, Cytomegalovirus (IgG; IgM)-Antikörpern oder Hepatitis B-Oberflächen-Antigen ($HB_sAg$) in Seren mittels Mikrotitrationsplatten, an denen entsprechend Rubella-Antigen, Cytomegalovirus-Antigen oder Antikörper gegen $HB_sAg$ fixiert sind, bestimmt wird.

8. Verfahren nach Anspruch 1, dadurch gekennzeichnet, das alkalische Phosphatase oder Peroxidase zur Herstellung der Farblösung eingesetzt wird.

FIG. 1

"Einpunktmessung" nach
van Loon

A,B,C positive Seren
D negatives Serum

0 264 866

0 264 866

FIG.2

spez.Farbsignal $A_{0D}$

lg $T_{mess}$

FIG.3

FIG.4